# EUROPÄISCHE PATENTANMELDUNG

(11) **EP 1 488 814 A1**
(43) Veröffentlichungstag der Anmeldung: **22.12.2004**
(21) Anmeldenummer: 04014400.8
(22) Anmeldetag: 18.06.2004
(51) Int. Cl.: A61L 9/12

(54) **Beduftungsgerät mit automatischer Steuerung**

(30) Priorität: 20.06.2003 DE 20309534 U
(71) Anmelder: Aromata International GmbH, 87477 Sulzberg (DE)
(72) Erfinder: Nübling, Kurt Ludwig, 87477 Sulzberg (DE)
(74) Vertreter: Barz, Peter, Dr.

(57) **Zusammenfassung**

Beduftungsgerät zur automatischen Steuerung der Verteilung eines Duftstoffs umfassend ein Speichermaterial für den Duftstoff oder eine Einrichtung zur Aufnahme eines derartigen Speichermaterials und einen Ventilator in einem Gehäuse mit mindestens einer Austrittsöffnung und einen Spender für den Duftstoff umfassend einen Behälter, einen Fördermechanismus und eine Steuereinheit für den Fördermechanismus, wobei im Behälter befindlicher Duftstoff mit Hilfe des Fördermechanismus auf im Gehäuse befindliches Speichermaterial aufgebracht werden kann und die Aufbringung automatisch von der Steuereinheit steuerbar ist.

## Beschreibung

Die Erfindung betrifft ein Beduftungsgerät, das für eine einfach ausführbare, automatisierte Beduftung von Räumen geeignet ist. Das Beduftungsgerät wird vorzugsweise zur Beduftung mit ätherischen Ölen verwendet.

Herkömmlicherweise werden Räume beduftet, indem der Duftstoff in ein offenes Gefäß gegeben und dann erwärmt wird. Eine andere Möglichkeit ist die Aufnahme des Duftstoffs auf ein Speichermaterial und anschließende Verteilung in die Umgebung mit Hilfe eines Ventilators. Bei beiden Verfahren muss die Wiederauffüllung manuell erfolgen. Es sind auch Sprühverfahren zur Beduftung bekannt, die automatisierbar sind. Geeignete Sprühvorrichtungen sind aber relativ komplex.

Die Aufgabe der vorliegenden Erfindung besteht in der Bereitstellung einer Beduftungsvorrichtung, die eine möglichst einfache und automatisierte Beduftung ermöglicht.

Diese Aufgabe wird erfindungsgemäß mit einem Beduftungsgerät gelöst, welches umfasst:
a) ein Speichermaterial für den Duftstoff oder eine Einrichtung zur Aufnahme eines derartigen Speichermaterials 3 und einen Ventilator in einem Gehäuse 1 mit mindestens einer Austrittsöffnung 4 und
b) einen Spender für den Duftstoff umfassend einen Behälter 2, einen Fördermechanismus 5 und eine Steuereinheit für den Fördermechanismus,
wobei im Behälter befindlicher Duftstoff mit Hilfe des Fördermechanismus auf im Gehäuse befindliches Speichermaterial aufgebracht werden kann und die Aufbringung automatisch von der Steuereinheit steuerbar ist.

Bei den einsetzbaren Speichermaterialien handelt es sich um Materialien, die einerseits den Duftstoff in sich aufnehmen können, aber andererseits relativ leicht wieder abgeben können, z.B. bei Einwirkung eines Luftstroms. Das Speichermaterial weist bevorzugt Rückhaltevermögen und ein relativ großes Aufnahmevermögen für den Duftstoff auf. Das Rückhaltevermögen kann aber überwunden werden, so dass das Speichermaterial über einen bestimmten Zeitraum den gespeicherten Duftstoff wieder abgibt. Die Abgabemenge pro Zeiteinheit hängt von der Stärke des Luftstroms ab.

Beispiele für einsetzbare Speichermaterialien sind Textilien, Papier, Watte oder Schaumstoffe. Bevorzugte Textilien sind Textilverbundstoffe, wie z.B. Filze und Vliese bzw. Vliesstoffe. Besonders bevorzugt als Speichermaterial ist ein Vlies.

Die anderen Komponenten des Beduftungsgeräts können aus geeigneten bekannten Materialien gebildet werden, z.B. Kunststoff, Glas oder Metall.

Das Beduftungsgerät ist vorzugsweise so gestaltet, dass das Speichermaterial bequem auswechselbar ist. Der Platz 3 im Gehäuse 1, der für das Speichermaterial vorgesehen ist, befindet sich vorzugsweise im unteren Teil des Gehäuses, z.B. am Boden. Es kann zweckmäßig sein, das Speichermaterial im Gehäuse zu fixieren, z.B. mit einer Fixiereinrichtung oder einer geeigneten Formgebung des Gehäuses, um eine stabile Lage des Speichermaterials im Gehäuse zu gewährleisten. Es kann auch sinnvoll sein, den Platz für das Speichermaterial so zu gestalten, dass gegebenenfalls vorhandener Duftstoff, der auf das Speichermaterial aufgebracht, aber noch nicht aufgenommen wurde, nicht ablaufen kann, um den Duftstoff vollständig auszunutzen und Verunreinigungen zu vermeiden.

Das Gehäuse bzw. der Korpus 1 kann ein- oder mehrteilig sein. Es kann z.B. zweckmäßig sein, dass der obere Teil des Gehäuses vom unteren abnehmbar ist, oder umgekehrt; um das Speichermaterial einzulegen oder auszuwechseln. Das Gehäuse kann auch einen schubladenartigen Mechanismus umfassen. Gewöhnlich besteht das Gehäuse aus Kunststoff. Die Austrittsöffnung(en) 4 sind gewöhnlich seitlich oder oben am Gehäuse angebracht. Zweckmäßigerweise handelt es sich um einen oder mehrere Lüftungsschlitze.

Der Ventilator wird relativ zum Speichermaterial so angeordnet, dass er einen Luftstrom erzeugen kann, der im Speichermaterial enthaltenen Duftstoff ausbläst und durch die mindestens eine Austrittsöffnung in die Umgebung verteilt. In der Regel ist der Ventilator über dem Speichermaterial angeordnet.

Der Ventilator kann z.B. über einen Ein/Aus-Schalter als Steuereinheit manuell in Betrieb genommen werden. Der Ventilator kann alternativ über eine Steuereinheit automatisch betätigt werden, wobei der Betrieb des Ventilators mit der Steuerung des Spenders abgestimmt werden kann. Die Steuerung des Ventilators kann von einer gesonderten Steuereinheit oder von der Steuereinheit für den Fördermechanismus übernommen werden. Die Steuereinheit für den Ventilator kann bezüglich der Drehzahl eine oder mehrere Stufen aufweisen oder stufenlos einstellbar sein.

Der Spender kann innerhalb oder außerhalb des Gehäuses angeordnet sein und umfasst einen Behälter 2, in den Duftstoff eingefüllt werden kann. Bei dem Duftstoff-Behälter handelt es sich z.B. um übliche Flaschen aus Glas oder Kunststoff.

Bei dem Fördermechanismus 5 handelt es sich um eine herkömmliche, dem Fachmann bekannte Fördereinrichtung, mit der Duftstoff aus dem Behälter befördert werden kann. Es handelt sich z. B. um einen Pumpverschluss, der auf dem Behälter angebracht ist. Der Fördermechanismus ist z.B. zweckmäßigerweise ein Pumpmechanismus oder ein Druckmechanismus, bei dem zur Förderung des Duftstoffs ein Druck aufgebaut wird. Der Fördermechanismus kann auch eine Zufuhrleitung umfassen, über die der Duftstoff vom Behälter auf im Gehäuse befindliches Speichermaterial aufgebracht werden kann.

Eine solche Zufuhrleitung ist am Behälter oder am Fördermechanismus angebracht und kann den geförderten bzw. abgepumpten Duftstoff zum Speichermaterial leiten, so dass der Duftstoff auf das Speichermaterial aufgebracht oder aufgeträufelt wird. Als Material für die Leitung eignet sich z.B. Glas oder Kunststoff.

Der Fördermechanismus und damit die Aufbringung des Duftstoffs auf das Speichermaterial werden über die Steuereinheit automatisch gesteuert. Die Steuereinheit ist einstellbar. Vorzugsweise umfasst die Steuereinheit eine Zeitsteuerung und/oder eine Mengensteuerung. Durch die Steuereinheit kann eine automatische Steuerung nach Bedarf programmiert werden.

Über eine Zeitautomatik können z.B. Intervall und Dauer des Betriebs eingestellt werden. Start- und Endzeiten können gewählt werden. Eine intermittierende Betriebsweise ist möglich. Mit einer Mengensteuerung lässt sich die Abgabemenge einstellen. Entsprechend kann der Ventilator gegebenenfalls automatisch gesteuert werden, z.B. bezüglich der Drehzahl und insbesondere der Zeit. Die Steuerung von Fördermechanismus und Ventilator können abgestimmt werden. Natürlich umfasst das Beduftungsgerät ferner gegebenenfalls notwendige elektrische Anschlusselemente, Schalter oder Regler.

Mit dem Beduftungsgerät ist eine automatisierte Beduftung von Räumen möglich. Im Spender kann ein Vorrat an Duftstoff eingefüllt werden, der bezüglich des Zeittakts und der Duftstoffmenge automatisch gesteuert auf das Speichermaterial aufgebracht wird, von dem es mit Hilfe des Ventilators in die Umgebung ausgeblasen wird. Die Steuerung kann individuell eingestellt werden. Es ist nur ein minimaler Arbeitsaufwand erforderlich, da die manuelle Nachfüllung des Speichermaterials entfällt.

Eine kompakte Konstruktion ist möglich. Es können z.B. Geräte mit einer Höhe von etwa 15 cm und einem Durchmesser von etwa 10 cm oder kleiner gebaut werden, so dass die Geräte als Tischgeräte verwendet werden können. Nach Bedarf kann das Gerät auch in größeren Dimensionen angefertigt werden, falls dies bei der Beduftung von größeren Räumen zweckdienlich ist.

Der Duftstoff kann in reiner Form oder gelöst in einem Lösungsmittel eingesetzt werden. Bevorzugt werden ätherische Öle, insbesondere unverdünnt, zur Beduftung verwendet.

Das Beduftungsgerät kann zur Beduftung von Räumen und auch von Großräumen, verwendet werden. Beispiele sind Privatwohnungen, Hotels, Kaufhäuser, Messehallen, Museen, Büroräume, Fabriken, Flughäfen, Sporthallen, Festsäle und Ausstellungsräume. Die Geräte sind vor allem im Business-to-Business- und Event-Sektor gut einsetzbar.

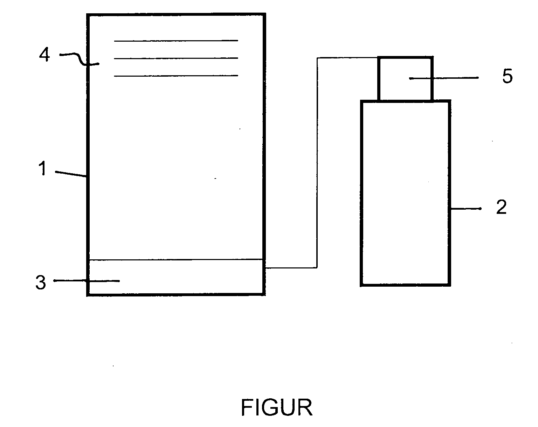

Ein Beispiel für eine Ausführungsform ist in der Figur veranschaulicht. Danach befindet sich in dem Gehäuse 1 ein Ventilator (nicht gezeigt). Im oberen Teil des Gehäuses 1 befinden sich Lüftungsschlitze 4, im unteren Teil ist eine Einrichtung zur Aufnahme von einem Vlies 3 vorgesehen. Die Einrichtung 3 kann abgenommen bzw. geöffnet werden, so dass das Vlies auswechselbar ist. Der Spender umfasst einen Behälter 2 und einen Fördermechanismus 5, bei dem es sich um einen Druckmechanismus handelt. Der Spender umfasst auch die Steuereinheit (nicht gezeigt), womit der Duftstoff automatisch vom Spender über die Zufuhrleitung auf das im Gehäuse befindliche Speichermaterial geträufelt wird.

## Patentansprüche

1. Beduftungsgerät zur automatischen Steuerung der Verteilung eines Duftstoffs umfassend
ein Speichermaterial für den Duftstoff oder eine Einrichtung zur Aufnahme eines derartigen Speichermaterials (3) und einen Ventilator in einem Gehäuse (1) mit mindestens einer Austrittsöffnung (4) und
einen Spender für den Duftstoff umfassend einen Behälter (2), einen Fördermechanismus (5) und eine Steuereinheit für den Fördermechanismus,
wobei im Behälter befindlicher Duftstoff mit Hilfe des Fördermechanismus auf im Gehäuse befindliches Speichermaterial aufgebracht werden kann und die Aufbringung automatisch von der Steuereinheit steuerbar ist.

2. Beduftungsgerät nach Anspruch 1, **dadurch gekennzeichnet, dass** das Speichermaterial auswechselbar ist.

3. Beduftungsgerät nach Anspruch 1 oder Anspruch 2, **dadurch gekennzeichnet, dass** die Steuereinheit eine Zeitsteuerung und/oder eine Mengensteuerung für die Aufbringung des Duftstoffs auf das Speichermaterial umfasst, die einstellbar sind.

4. Beduftungsgerät nach einem der Ansprüche 1 bis 3, **dadurch gekennzeichnet, dass** das Speichermaterial im unteren Teil des Gehäuses platziert ist oder dort platziert werden kann.

5. Beduftungsgerät nach einem der Ansprüche 1 bis 4, **dadurch gekennzeichnet, dass** der Ventilator in dem Gehäuse über dem Speichermaterial oder dem dafür vorgesehenen Platz angeordnet ist.

6. Beduftungsgerät nach einem der Ansprüche 1 bis 5, **dadurch gekennzeichnet, dass** die mindestens eine Austrittsöffnung Lüftungsschlitze sind.

7. Beduftungsgerät nach einem der Ansprüche 1 bis 6, **dadurch gekennzeichnet, dass** das Speichermaterial ein Vlies ist.

8. Beduftungsgerät nach einem der Ansprüche 1 bis 7, **dadurch gekennzeichnet, dass** die Steuereinheit auch den Ventilator steuert oder eine zusätzliche Steuereinheit zur Steuerung des Ventilators vorhanden ist.

9. Beduftungsgerät nach einem der Ansprüche 1 bis 8, **dadurch gekennzeichnet, dass** sich der Spender innerhalb oder außerhalb des Gehäuses befindet.

10. Beduftungsgerät nach einem der Ansprüche 1 bis 9, **dadurch gekennzeichnet, dass** der Fördermechanismus einen Pumpmechanismus oder Druckmechanismus umfasst.
